# EUROPEAN PATENT SPECIFICATION

(11) **EP 4 483 850 B1**
(45) Date of publication and mention of the grant of the patent: **10.06.2026**
(21) Application number: 24208746.8
(22) Date of filing: 08.01.2021
(51) Int. Cl.: A61F 2/24, A61F 2/915

(54) **PROSTHETIC HEART VALVE LEAFLET ASSEMBLIES AND METHODS**
PROTHETISCHE HERZKLAPPENSEGELANORDNUNGEN UND VERFAHREN
ENSEMBLES FEUILLETS DE VALVULE CARDIAQUE PROTHÉTIQUE ET PROCÉDÉS

(30) Priority: 14.01.2020 US 202062960838 P
(43) Date of publication of application: 01.01.2025
(62) Divisional of application: 21703119.4
(73) Proprietor: Edwards Lifesciences Corporation, Irvine, CA 92614 (US)
(72) Inventor: TZADOK, Sara, 30600 Or-akiva (IL); NIR, Noam, 30889 Caesarea (IL)
(74) Representative: Venner, Julia Ann

(56) References cited:
- US-A1- 2004 039 436
- US-A1- 2011 295 363
- US-A1- 2018 028 310
- US-A1- 2019 159 895
- US-B2- 9 510 942

## Description

### CROSS REFERENCE TO RELATED APPLICATIONS

This application claims the benefit of U.S. Provisional Application 62/960,838 filed on January 14, 2020.

### FIELD

The present disclosure relates to prosthetic heart valves, and to methods and assemblies for forming commissures associated with leaflets of such prosthetic heart valves.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery device and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery device so that the prosthetic valve can self-expand to its functional size.

Prosthetic valves that rely on a mechanical actuator for expansion can be referred to as "mechanically expandable" prosthetic heart valves. The actuator typically takes the form of pull cables, sutures, wires and/or shafts that are configured to transmit expansion forces from a handle of the delivery apparatus to the prosthetic valve. Most expandable, transcatheter heart valves comprise a cylindrical metal frame or stent and prosthetic leaflets mounted inside the frame. The leaflets may be attached to the frame at commissure tab of the leaflets. However, in some configurations, forces experienced during operation of the prosthetic valve and/or other conditions may be concentrated at the commissure tabs, which may compromise the structure of the leaflets and/or cause the leaflets to detach from the frame. Furthermore, the attachment of the commissure tabs to the frame in such configurations may require delicate assembly skills to ensure proper attachment and reduce damage to the leaflets.

US 2019/159895 describes a radially collapsible and expandable prosthetic heart valve. The prosthetic valve can comprise an annular frame, leaflets, an inner skirt, and an outer skirt.

US 2004/039436 describes a valve prosthesis device suitable for implantation in body ducts. The device comprises a support stent, comprised of a deployable construction adapted to be initially crimped in a narrow configuration suitable for catheterization through the body duct to a target location and adapted to be deployed by exerting substantially radial forces from within by means of a deployment device to a deployed state in the target location, and a valve assembly comprising a flexible conduit having an inlet end and an outlet, made of pliant material attached to the support beams providing collapsible slack portions of the conduit at the outlet. The support stent is provided with a plurality of longitudinally rigid support beams of fixed length.

US 2011/295363 describes a prosthetic heart valve for an endoprosthesis used in the treatment of a stenotic cardiac valve and/or a cardiac valve insufficiency. The prosthetic heart valve comprises of a plurality of leaflets, which consist of a natural and/or synthetic material and have a first opened position for opening the heart chamber and a second closed position for closing the heart chamber, the leaflets being able to switch between their first and second position in response to the blood flow through the heart. In addition, the prosthetic heart valve comprises a leaflet support portion, consisting of biological and/or synthetic material for mounting of the prosthetic heart valve to a stent, and a bendable transition area which forms a junction between the leaflets and the leaflet support portion, the transition area progressing essentially in a U-shaped manner similar to a cusp shape of a natural aortic or pulmonary heart valve for reducing tissue stresses during opening and closing motion of the leaflets.

US 9 510 942 describes an implantable prosthetic valve which has an upper frame section and a lower frame section. The upper frame section has a plurality of struts and a first leaflet receiving surface at a lower portion of the upper frame section. The lower frame section has a second leaflet receiving surface at an upper portion of the lower frame section. An edge of a flexible leaflet is disposed between the first and second leaflet receiving surfaces to attach the leaflet to the upper and lower frame sections.

US 2018/028310 describes a prosthetic heart valve which can include a radially collapsible and expandable annular frame having a plurality of struts defining openings. The prosthetic heart valve can further include a plurality of leaflets that regulate the flow of blood through the frame. The prosthetic heart valve can also include a sealing member mounted on the frame and having an inner layer and an outer layer.

Accordingly, a need exists for improved prosthetic heart valve leaflet assemblies and methods for assembling the leaflet assemblies to a frame of the prosthetic heart valve.

### SUMMARY

Aspects of the presently claimed invention are set out in the independent claims. Particular embodiments of these aspects are set out in the dependent claims. Any subject matter contained herein that does not fall within the scope of the appended claims is considered as being useful for understanding the invention.

Described herein are embodiments and examples of methods for assembling a prosthetic heart valve, methods of assembling a commissure of a prosthetic valve, and a prosthetic heart valve including a plurality of commissures. In some embodiments and examples, the commissures may be formed by coupling a pair of adjacent commissure tabs of adjacent leaflets of the prosthetic heart valve. The commissures may include a support strip, optionally folded over an inner reinforcing element, with a main body and a tab portion extending from and folded over a portion of the main body. The support strip may include alignment indicators for alignment of the tab portion over the main body, and alignment markings for locations of sutures that pass through the support strip and other elements of the commissure to form the commissure and/or secure the commissure to a support structure of the prosthetic heart valve.

In one example, a method of assembling a prosthetic heart valve comprising a plurality of leaflets can include: forming a plurality of commissures with the plurality of leaflets, wherein each commissure is formed by pairing a first commissure tab of a first leaflet with an adjacent, second commissure tab of a second leaflet, coupling a main body of a support strip to the first and second commissure tabs via primary sutures, folding a tab portion of the support strip over the main body, and coupling the tab portion of the support strip to the main body via secondary sutures, and, for each commissure, securing the commissure to a respective support portion of a frame of the prosthetic heart valve.

In another example, a prosthetic heart valve can include: an annular frame comprising a plurality of commissure support portions, and a plurality of leaflets, each leaflet having a commissure tab that is coupled to an adjacent commissure tab of another leaflet via a support strip to form a commissure of an associated commissure tab pair, and, for each commissure tab pair: the support strip includes a main body and a tab portion extending from and folded over a portion of the main body, a primary suture passes through, in order or in reverse order, a first side of the main body, a first commissure tab of the commissure tab pair, a second commissure tab of the commissure tab pair, and a second side of the main body, and a secondary suture passes through the tab portion and the main body to couple the tab portion to the main body, and each commissure is secured to a corresponding commissure support portion of the plurality of commissure support portions.

In yet another example, a method of assembling a commissure for a leaflet pair of a prosthetic valve can include: attaching a main body of a support strip to adjacent ends of a first commissure tab of a first leaflet of the leaflet pair and a second commissure tab of a second leaflet of the leaflet pair by stitching primary sutures through edge alignment markings of the support strip, folding and aligning a tab portion of the support strip over the main body of the support strip in accordance with one or more strip alignment indicators, attaching the tab portion of the support strip to the main body of the support strip by stitching secondary sutures through central alignment markings of the support strip, and coupling end portions of the main body of the support strip to one another around a support portion of a frame of the prosthetic valve via tertiary sutures to secure the commissure to the support portion of the frame.

The foregoing and other objects, features, and advantages of the invention will become more apparent from the following detailed description, which proceeds with reference to the accompanying figures.

### BRIEF DESCRIPTION OF THE DRAWINGS

FIG. 1 is a perspective view of an exemplary embodiment of a prosthetic heart valve.
FIG. 2A illustrates a first view of an example commissure of a prosthetic valve in accordance with the presently claimed invention, in which the commissure is secured to a support post of the prosthetic valve.
FIG. 2B illustrates a second view of the assembled commissure of FIG. 2A.
FIG. 3A illustrates a cross-sectional view of the example assembled commissure of FIG. 2A in a first state.
FIG. 3B illustrates a cross-sectional view of the example assembled commissure of FIG. 2A in a second state.
FIG. 4 illustrates an example schematic representation of an assembled commissure having a support strip with a folded-up tab portion.
FIG. 5 illustrates an example of an unfolded support strip for use in reinforcing a commissure of a prosthetic valve, where the support strip includes alignment notches.
FIG. 6 illustrates the support strip of FIG. 5 after folding a tab of the support strip over a main body of the support strip.
FIG. 7 illustrates another example of a support strip that includes alignment markings or apertures instead of alignment notches.
FIG. 8 illustrates another example of a support strip that includes a single line of alignment markings or apertures on a tab of the support strip.
FIG. 9 is a flow chart of an example method of assembling a commissure of a prosthetic valve and securing the commissure to a frame of the prosthetic valve.
FIG. 10 is a side elevation view of a delivery apparatus for a prosthetic heart valve, according to one example.

### DETAILED DESCRIPTION

### General Considerations

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved. The technologies from any example can be combined with the technologies described in any one or more of the other examples. In view of the many possible embodiments to which the principles of the disclosed technology may be applied, it should be recognized that the illustrated embodiments are only preferred examples and should not be taken as limiting the scope of the disclosed technology.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used herein, with reference to the prosthetic heart valve and the transcatheter delivery system, "proximal" refers to a position, direction, or portion of a component that is closer to the user and a handle of the delivery system that is outside the patient, while "distal" refers to a position, direction, or portion of a component that is further away from the user and the handle and closer to the implantation site. The terms "longitudinal" and "axial" refer to an axis extending in the proximal and distal directions, unless otherwise expressly defined.

As used in this application and in the claims, the singular forms "a," "an," and "the" include the plural forms unless the context clearly dictates otherwise. Additionally, the term "includes" means "comprises." Further, the terms "coupled" and "connected" generally mean electrically, electromagnetically, and/or physically (e.g., mechanically or chemically) coupled or linked and does not exclude the presence of intermediate elements between the coupled or associated items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or," as well as "and" and "or."

### Examples of the Disclosed Technology

Described herein are examples of prosthetic heart valves, commissures for prosthetic valves, and methods for assembling commissures of prosthetic valves. The prosthetic heart valves may include a frame and a plurality of leaflets attached to the frame via commissures formed by joining pairs of adjacent ends (e.g., commissure tabs) of the leaflets. The formation of the commissures may include attaching a support strip (e.g., including a main portion and a tab portion folded over a central region of the main portion), which is optionally folded over one or more reinforcing members (e.g., in alignment according to one or more alignment indicators on the support strip), to commissure tabs of the leaflets. Opposing ends of the support strip may be coupled together via a primary suture(s) passing through the opposing ends of the support strip (e.g., in locations corresponding to alignment markers forming suture lines on the support strip), the commissure tabs, and optionally the reinforcing members. The folded-over tab portion of the support strip may be secured to the main portion of the support strip via one or more secondary sutures each passing through the tab portion of the support strip (e.g., in locations corresponding to alignment markers forming suture lines on the support strip), a respective commissure tab, a respective side of the main portion of the support strip, and optionally a respective reinforcing member. Opposing ends of the primary suture(s), secondary sutures, and/or an additional suture(s) (e.g., tertiary sutures) may be wrapped around a corresponding commissure support portion of the frame of the prosthetic valve to secure the commissure to the frame.

In this way, forces experienced by the leaflets during radial expansion and compression of the frame and/or as the leaflets open and closed during operation of the prosthetic valve may be at least partially absorbed by the support strip, reducing the stresses exerted on the leaflets. As the support strip may be made of more robust material than the leaflets, the overall strength of the commissure may be increased relative to other configurations. Folding a portion of the support strip over itself (e.g., folding a tab portion of the support strip over a central region of a main body of the support strip) provides additional reinforcing strength for the commissure by providing a further deflection of stresses away from the potentially relatively delicate material of the leaflets. The use of alignment indicators (e.g., notches, markings, apertures, etc.) and/or alignment markings forming suture lines provides additional guidance during assembly of the commissure, reducing assembly difficulty and ensuring proper alignment of elements to maximize reinforcement strength.

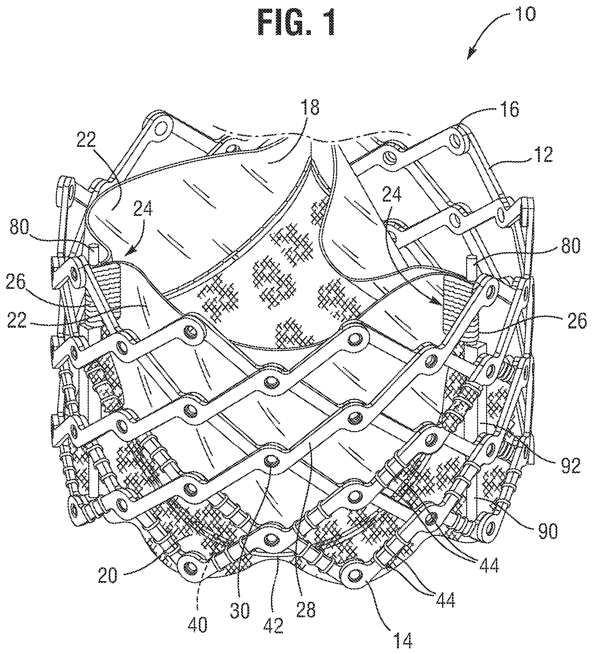

FIG. 1 shows an exemplary prosthetic heart valve 10, according to one embodiment. The prosthetic heart valve 10 can be radially compressible and expandable between a radially compressed configuration for delivery into a patient and a radially expanded configuration. In particular embodiments, the prosthetic heart valve 10 can be implanted within the native aortic valve, although it also can be implanted at other locations in the heart, including within the native mitral valve, the native pulmonary valve, and the native tricuspid valve. The prosthetic heart valve 10 can include an annular stent or frame 12 having a first end 14 and a second end 16.

In the depicted embodiment, the first end 14 is an inflow end and the second end 16 is an outflow end. The outflow end 16 can be coupled to a delivery apparatus for delivering and implanting the prosthetic heart valve within the native aortic valve is a transfemoral, retrograde delivery approach. Thus, in the delivery configuration of the prosthetic heart valve, the outflow end 16 is the proximal-most end of the prosthetic valve. In other embodiments, the inflow end 14 can be coupled to the delivery apparatus, depending on the particular native valve being replaced and the delivery technique that is used (e.g., trans-septal, transapical, etc.). For example, the inflow end 14 can be coupled to the delivery apparatus (and therefore is the proximal-most end of the prosthetic heart valve in the delivery configuration) when delivering the prosthetic heart valve to the native mitral valve via a trans-septal delivery approach.

The frame 12 can be made of any of various suitable materials, such as stainless steel, a cobalt chromium alloy, or a nickel titanium alloy ("NiTi"), for example Nitinol. Referring again to FIG. 1, as shown, the frame 12 can include a plurality of interconnected struts 28 arranged in a lattice-type pattern. The struts 28 are shown as positioned diagonally, or offset at an angle relative to, and radially offset from, a longitudinal axis of the prosthetic heart valve 10 when the prosthetic heart valve 10 is in the expanded configuration. In other implementations, the struts 28 can be offset by a different amount than depicted in FIG. 1, or some or all of the struts 28 can be positioned parallel to the longitudinal axis of the prosthetic heart valve 10.

In the illustrated embodiment, the struts 28 are pivotably coupled to one another at one or more pivot joints along the length of each strut. For example, in the illustrated configuration, each of the struts 28 can be formed with apertures at opposing ends of the strut and apertures spaced along the length of the strut. Respective hinges can be formed at the locations where struts 28 overlap each other via fasteners or pivot members, such as rivets or pins 30 that extend through the apertures. The hinges can allow the struts 28 to pivot relative to one another as the frame 12 is radially expanded or compressed, such as during assembly, preparation, or implantation of the prosthetic heart valve 10.

In some embodiments, the frame 12 can be constructed by forming individual components (e.g., the struts and fasteners of the frame) and then mechanically assembling and connecting the individual components together. In other embodiments, the struts 28 are not coupled to each other with respective hinges but are otherwise pivotable or bendable relative to each other to permit radial expansion and contraction of the frame 12. For example, the frame 12 can be formed (e.g., via laser cutting, electroforming or physical vapor deposition) from a single piece of material (e.g., a metal tube). Further details regarding the construction of the frame and the prosthetic heart valve are described in U.S. Patent Application Publication Nos. 2018/0153689, 2018/0344456, and 2019/0060057.

The prosthetic heart valve 10 can also include a valvular structure 18 which is coupled to the frame 12 and configured to regulate the flow of blood through the prosthetic heart valve 10 from the inflow end 14 to the outflow end 16. The prosthetic heart valve 10 can further include a plurality of actuators 80 mounted to and equally spaced around the inner surface of the frame 12. The actuators are configured to apply expansion and compression to the frame for radially expanding and compressing the prosthetic valve.

In the illustrated embodiment, the actuators 80 are linear actuators, each of which comprises an inner member, or piston, 90 and an outer member, or cylinder, 92. The inner member 90 is pivotably coupled to a junction of the frame, such as at the first end 14, while the outer member 92 is pivotably coupled to another junction of the frame closer to the second end 16. Moving the inner member 90 proximally relative to the outer member 92 and/or moving the outer member 92 distally relative to the inner member 90 is effective to radially expand the prosthetic valve. Conversely, moving the inner member 90 distally relative to the outer member 92 and/or moving the outer member 92 proximally relative to the inner member 90 is effective to radially compress the prosthetic valve. The actuators 80 can include locking mechanisms that are configured to retain the prosthetic valve in an expanded state inside the patient's body.

In some embodiments, each of the actuators 80 can be configured to form a releasable connection with one or more respective actuators of a delivery apparatus of a transcatheter delivery system. The actuators of the delivery apparatus can transmit forces from a handle of the delivery apparatus to the actuators 80 for expanding or compressing the prosthetic valve. Further details of the actuators, locking mechanisms and delivery apparatuses for actuating the actuators can be found in U.S. Patent Application Publication Nos. 2018/0153689, 2019/0060057 and 2018/0325665, PCT Application No. PCT/US2020/057691, filed October 28, 2020, PCT Application No. PCT/US2020/063104, filed December 3, 2020, and U.S. Application No. 62/990,299, filed March 16, 2020. Any of the actuators and locking mechanisms disclosed in the previously filed applications can be incorporated in any of the prosthetic valves disclosed herein. Further, any of the delivery apparatuses disclosed in the previously filed applications can be used to deliver and implant any of the prosthetic valves discloses herein.

In some embodiments, each of the actuators 80 can be used to support a respective commissure 24 (described below). As such, the actuators 80 can include commissure support portions for supporting and attaching commissures 24 of the valvular structure 18 to the frame 12, as described further herein.

The valvular structure 18 can include, for example, a leaflet assembly comprising one or more leaflets 22 (three leaflets 22 in the illustrated embodiment) made of a flexible material. The leaflets 22 of the leaflet assembly can be made from in whole or part, biological material, bio-compatible synthetic materials, or other such materials. Suitable biological material can include, for example, bovine pericardium (or pericardium from other sources). The leaflets 22 can be arranged to form commissures 24, which can be, for example, mounted to commissure support portions of respective actuators 80. Further details regarding transcatheter prosthetic heart valves, including the manner in which the valvular structure can be coupled to the frame 12 of the prosthetic heart valve 10, can be found, for example, in U.S. Patent Nos. 6,730,118, 7,393,360, 7,510,575, 7,993,394, and 8,652,202, and U.S. Patent Application Publication No. 2018/0325665.

In some embodiments, as shown in FIG. 1, the commissures 24 can be mounted (e.g., sutured) directly to commissure support portions of the actuators 80 of the frame 12 via commissure attachments 26, which can be strips of fabric. As one example, each commissure attachment 26 may be wrapped around a corresponding actuator 80 and a pair of adjacent commissure tabs of adjacent leaflets and secured to the commissure tabs and the actuator via one or more stitches extending through the commissure attachment and the pair of the commissure tabs. In other embodiments, the commissures 24 can be mounted to support struts or posts of the frame that are separate from the actuators 80.

The prosthetic heart valve 10 can also include one or more skirts or sealing members. For example, as shown in FIG. 1, the prosthetic heart valve 10 can include an inner skirt 20 mounted on the inner surface of the frame 12. As shown in FIG. 1, the inner skirt 20 is a circumferential inner skirt that spans an entire circumference of the inner surface of the frame 12. The inner skirt 20 can function as a sealing member to prevent or decrease paravalvular leakage (e.g., when the valve is placed at the implantation site) and as an attachment surface to anchor the leaflets 22 to the frame 12.

For example, as shown, a cusp edge portion 40 of each leaflet 22 (the inflow edge portion) can be secured to the inner skirt 20 with stitching 42 (referred to as a "scallop line"). The upper and lower edge portions of the inner skirt 20 can be secured to the frame with suture loops 44 that extending through the inner skirt and around adjacent struts 28 of the frame. In this manner, the cusp edge portions of the leaflets are supported by the inner skirt 20 and the commissures are supported by actuators 80.

The prosthetic heart valve 10 can also include an outer skirt mounted on the outer surface of the frame 12 (not shown in FIG. 1). The outer skirt can function as a sealing member for the prosthetic valve by sealing against the tissue of the native valve annulus and helping to reduce paravalvular leakage past the prosthetic valve. The inner and outer skirts can be formed from any of various suitable biocompatible materials, including any of various synthetic materials (e.g., PET) or natural tissue (e.g., pericardial tissue). The inner and outer skirts can be mounted to the frame using sutures, an adhesive, welding, and/or other means for attaching the skirts to the frame.

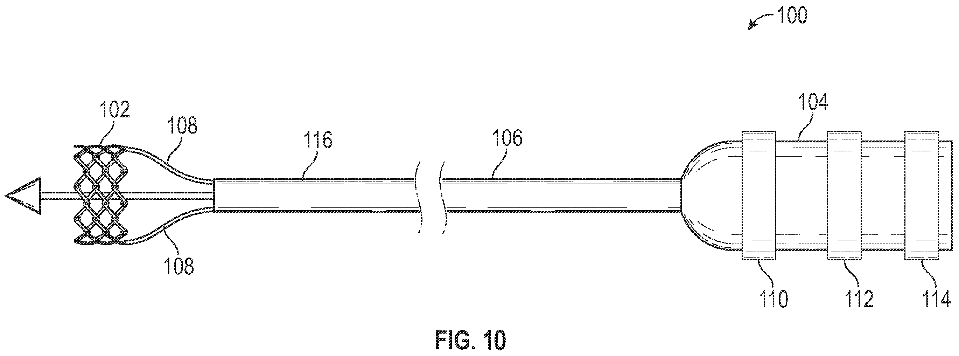

FIG. 10 illustrates a delivery apparatus 100, according to one embodiment, adapted to deliver a prosthetic heart valve 102, such as the illustrated prosthetic heart valve 10, described above with respect to FIG. 1. The prosthetic valve 102 can be releasably coupled to the delivery apparatus 100. It should be understood that the delivery apparatus 100 and other delivery apparatuses disclosed herein can be used to implant prosthetic devices other than prosthetic valves, such as stents or grafts.

The delivery apparatus 100 in the illustrated embodiment generally includes a handle 104, a first elongated shaft 106 (which comprises an outer shaft in the illustrated embodiment) extending distally from the handle 104, at least one actuator assembly 108 extending distally through the outer shaft 106. The at least one actuator assembly 108 can be configured to radially expand and/or radially collapse the prosthetic valve 102 when actuated.

Though the illustrated embodiment shows two actuator assemblies 108 for purposes of illustration, it should be understood that one actuator 108 can be provided for each actuator on the prosthetic valve. For example, three actuator assemblies 108 can be provided for a prosthetic valve having three actuators. In other embodiments, a greater or fewer number of actuator assemblies can be present.

In some embodiments, a distal end portion 116 of the shaft 106 can be sized to house the prosthetic valve in its radially compressed, delivery state during delivery of the prosthetic valve through the patient's vasculature. In this manner, the distal end portion 116 functions as a delivery sheath or capsule for the prosthetic valve during delivery,

The actuator assemblies 108 can be releasably coupled to the prosthetic valve 102. For example, in the illustrated embodiment, each actuator assembly 108 can be coupled to a respective actuator of the prosthetic valve 102. Each actuator assembly 108 can comprise a support tube, an actuator member, and a locking tool. When actuated, the actuator assembly can transmit pushing and/or pulling forces to portions of the prosthetic valve to radially expand and collapse the prosthetic valve as previously described. The actuator assemblies 108 can be at least partially disposed radially within, and extend axially through, one or more lumens of the outer shaft 106. For example, the actuator assemblies 108 can extend through a central lumen of the shaft 106 or through separate respective lumens formed in the shaft 106.

The handle 104 of the delivery apparatus 100 can include one or more control mechanisms (e.g., knobs or other actuating mechanisms) for controlling different components of the delivery apparatus 100 in order to expand and/or deploy the prosthetic valve 102. For example, in the illustrated embodiment the handle 104 comprises first, second, and third knobs 110, 112, and 114.

The first knob 110 can be a rotatable knob configured to produce axial movement of the outer shaft 106 relative to the prosthetic valve 102 in the distal and/or proximal directions in order to deploy the prosthetic valve from the delivery sheath 116 once the prosthetic valve has been advanced to a location at or adjacent the desired implantation location with the patient's body. For example, rotation of the first knob 110 in a first direction (e.g., clockwise) can retract the sheath 116 proximally relative to the prosthetic valve 102 and rotation of the first knob 110 in a second direction (e.g., counter-clockwise) can advance the sheath 116 distally. In other embodiments, the first knob 110 can be actuated by sliding or moving the knob 110 axially, such as pulling and/or pushing the knob. In other embodiments, actuation of the first knob 110 (rotation or sliding movement of the knob 110) can produce axial movement of the actuator assemblies 108 (and therefore the prosthetic valve 102) relative to the delivery sheath 116 to advance the prosthetic valve distally from the sheath 116.

The second knob 112 can be a rotatable knob configured to produce radial expansion and/or contraction of the prosthetic valve 102. For example, rotation of the second knob 112 can move the actuator member and the support tube axially relative to one another. Rotation of the second knob 112 in a first direction (e.g., clockwise) can radially expand the prosthetic valve 102 and rotation of the second knob 112 in a second direction (e.g., counter-clockwise) can radially collapse the prosthetic valve 102. In other embodiments, the second knob 112 can be actuated by sliding or moving the knob 112 axially, such as pulling and/or pushing the knob.

The third knob 114 can be a rotatable knob configured to retain the prosthetic heart valve 102 in its expanded configuration. For example, the third knob 114 can be operatively connected to a proximal end portion of the locking tool of each actuator assembly 108. Rotation of the third knob in a first direction (e.g., clockwise) can rotate each locking tool to advance the locking nuts to their distal positions to resist radial compression of the frame of the prosthetic valve, as described above. Rotation of the knob 114 in the opposite direction (e.g., counterclockwise) can rotate each locking tool in the opposite direction to decouple each locking tool from the prosthetic valve 102. In other embodiments, the third knob 114 can be actuated by sliding or moving the third knob 114 axially, such as pulling and/or pushing the knob.

Although not shown, the handle 104 can include a fourth rotatable knob operative connected to a proximal end portion of each actuator member. The fourth knob can be configured to rotate each actuator member, upon rotation of the knob, to unscrew each actuator member from the proximal portion of a respective actuator. As described above, once the locking tools and the actuator members are uncoupled from the prosthetic valve 102, they can be removed from the patient.

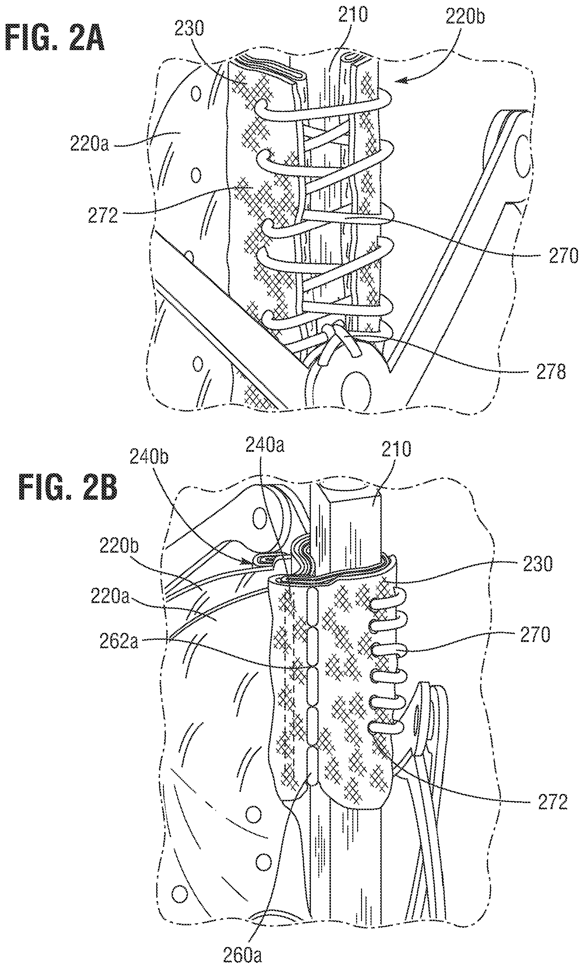

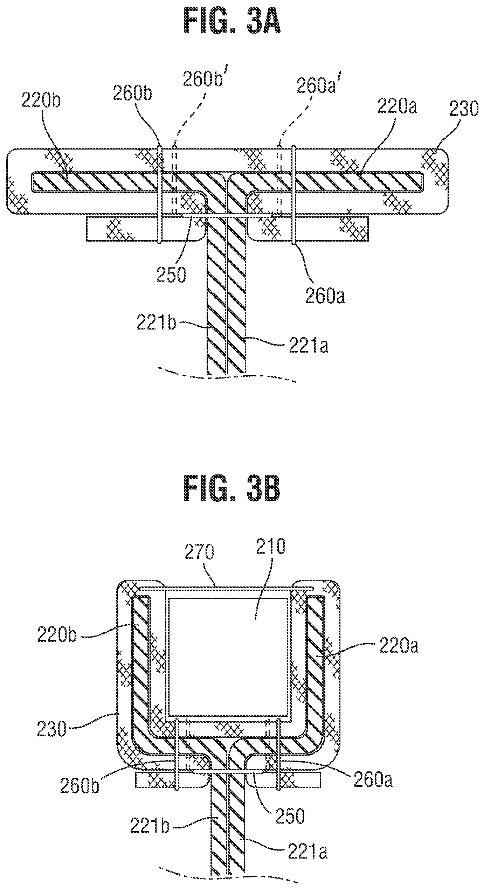

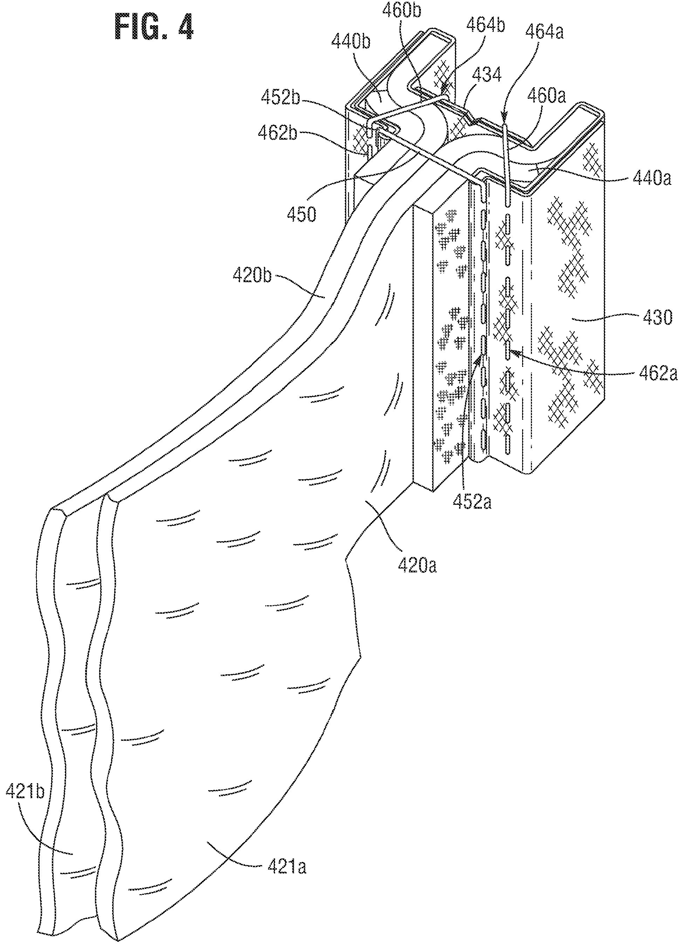

FIGS. 2A-4 show example commissure tab assemblies and attachments of commissure tab assemblies to a commissure post or other support structure of a frame of a prosthetic valve. FIGS. 2A and 2B show an example commissure tab assembly from two different view angles and FIGS. 3A and 3B show example cross-sectional views of the commissure tab assembly in two different states (e.g., prior to wrapping the commissure around a support structure of the frame and after wrapping the commissure at least partially around a support structure of the frame, respectively). FIG. 4 shows a schematic representation of a commissure tab assembly including elements corresponding to those of FIGS. 2A-3B.

A commissure tab assembly of the presently claimed invention is pre-assembled prior to attachment thereof to the frame by performing a pre-assembly process. The pre-assembly process includes extending a primary suture 250 along a first suture line having a plurality of in-and-out stitches extending through, in order (or in the reverse order, e.g., starting from the last-listed element and extending through the following list of elements, in reverse, to the first-listed element), a first side of a support strip (e.g., a flexible cloth/fabric) 230, optionally a first reinforcing element 240a (for example configurations that include reinforcing members/elements), a first commissure tab 220a, a second commissure tab 220b (where commissure tabs 220a and 220b are two commissure tabs of adjacent leaflets, e.g., leaflets 221a and 221b), optionally a second reinforcing element 240b (for example configurations that include reinforcing members), and along (e.g., through apertures/markings of) a second suture line on the second side of the support strip 230. As used herein, the term "suture line" can also be referred to as a "stitch line." The second side of the support is opposite the first side relative to a center of the support strip (e.g., a centerline that divides a width/longest dimension of the strip in half).

FIG. 4 shows another example of a primary suture 450 forming stitches passing from primary suture lines 452a (e.g., through edge alignment markings on a first side of a support strip 430) to primary suture lines 452b (e.g., through edge alignment markings on a second side of the support strip 430 opposite of the first side), where the suture 450 passes through one or more layers of the first side of the support strip 430, reinforcing elements 440a and 440b, commissure tabs 420a and 420b (of leaflets 421a and 421b, respectively), and one or more layers of the second side of the support strip 430.

Returning to FIGS. 2A-3B, the support strip 230 may be a strip of any suitable material (e.g., fabric), which may include material that is stronger (e.g., more resilient to tearing and/or deforming) than a material used for forming the leaflets and/or the commissure tab portions of the leaflets of the prosthetic valve. In one example, the support strip 230 is a polyethylene terephthalate (PET) fabric, although various other suitable biocompatible fabrics can be used.

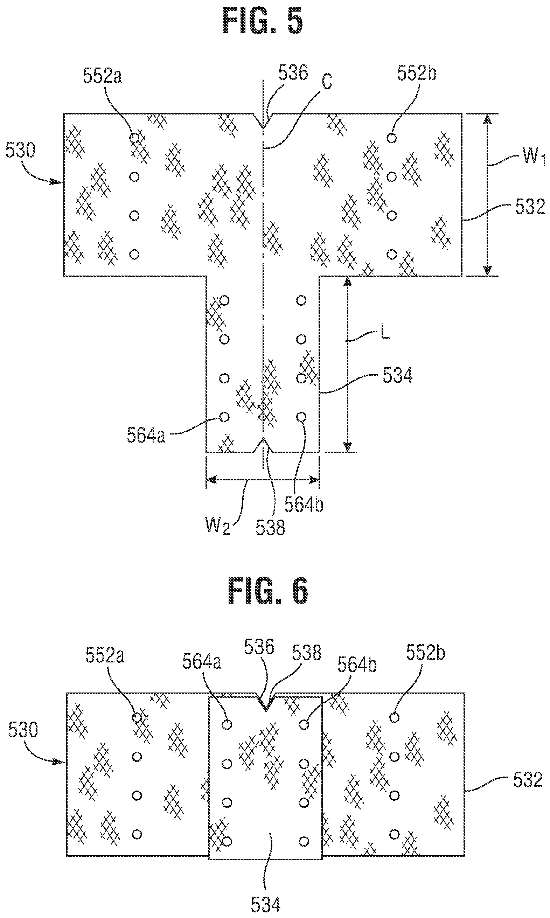

The support strip 230 may include a main body and a tab portion, as will be described in more detail below with respect to the example support strips illustrated in FIGS. 5-8, and the main body portion of the support strip may be longer in one dimension than another (e.g., having a width/length that is greater than a height of the strip). The support strip 230 may be continuous (e.g., with no gaps and/or having a substantially uniform distribution of the material forming the support strip, other than optional apertures forming the suture lines described herein) and may be relatively thin (e.g., having a thickness that is substantially smaller than the width and height of the strip and substantially smaller than a width or diameter of the optional reinforcing elements). Additional examples of features of the support strip 230 are described below with respect to FIGS. 5-8.

For example embodiments that include reinforcing elements, the reinforcing elements may include a string, a cord, and/or a relatively thick suture (e.g., an Ethibond suture), which may be substantially wider/thicker and/or have a substantially larger diameter (e.g., at least twice as wide/thick and/or have twice the diameter) than stitching sutures (e.g., primary sutures, secondary sutures, described in more detail below, tertiary sutures, described in more detail below, and/or other sutures of the commissure). The reinforcing elements may be substantially the same height (or slightly shorter, such as 1-5% shorter to provide for machining tolerances and avoid extension of the inner reinforcing element past edges of the strip) as the height of the support strip. In other examples, the reinforcing elements can be a relatively narrow strip(s) of fabric, which can be folded lengthwise one or more times to increase its overall thickness. In still other examples, the reinforcing elements can be a metal wire(s) or a bar(s), such as a rectangular or cylindrical bar(s), formed from a metal and/or a polymer.

The reinforcing elements may include two individual reinforcing elements or two portions of a single reinforcing member that extends along outer surfaces of each of the first and the second commissure tabs 220a/b (or 420a/b of FIG. 4). For example, a reinforcing member may be folded into a U-shape configuration to form the reinforcing elements (e.g., the reinforcing elements may be individual elements that are coupled to one another and/or form different sections of a single continuous element/member). In other examples, a reinforcing member may be discontinuous and include the reinforcing elements as discrete or separate elements in a spatially separated configuration (e.g., where a first reinforcing element is spatially separated from a second reinforcing element).

In some examples, the reinforcing elements may be aligned with one or more alignment markings and/or suture/stitching lines of the support strip. In some embodiments, the reinforcing elements are placed against outer surfaces of the commissure tabs, respectively, and opposing end or side portions of the support strip are positioned on an opposing side(s) of the reinforcing elements from the respective commissure tabs. For example, each reinforcing element may be sandwiched between a respective portion of the support strip and a respective one of the commissure tabs. In other embodiments, the support strip is at least partially wrapped or folded around the reinforcing elements to at least partially encase the reinforcing elements. In such examples, the portions of the support strip that at least partially encase the reinforcing elements may be placed against the outer surfaces of the commissure tabs, respectively.

Returning to the formation of the pre-assembled commissure in the presently claimed invention, additional, secondary sutures 260a and 260b are extended, each, in order (or in the reverse order): between inner secondary suture lines 262a and 262b (respectively), through the support strip 230, optionally through the respective reinforcing elements 240a and 240b (respectively, when included in the commissure), through the commissure tabs 220a and 220b (respectively) and finally along (e.g., through apertures/markings of) the outer secondary suture line 264a and 264b (respectively) of a mid-portion of the support strip 230.

FIG. 4 shows another example of secondary sutures 460a and 460b forming stitches passing from secondary suture lines 462a and 462b, respectively (e.g., through central alignment markings of the support strip 430), to secondary suture lines 464a and 464b, respectively (e.g., through central alignment markings of the support strip 430), where the sutures 460a and 460b each pass through one or more layers of the central region of the support strip 430, reinforcing elements 440a and 440b (respectively), commissure tabs 420a and 420b (respectively), and one or more layers of the end portions of the support strip 430 (respectively).

As will be described in more detail below with respect to FIGS. 5-8, the support strip may include two layers of material (e.g., a main body portion and a tab portion of the strip that is folded over a central region of the main body of the strip) between the commissure tabs and the support structure to which the commissure is to be attached. Accordingly, the sutures 260a and 260b of FIGS. 2A-3B may extend through both of the above-described layers of the support strip (e.g., the main body and the tab portion). For example, as shown in FIG. 4, sutures 460a and 460b extend through both the main body of the support strip 430 and a tab portion 434 of the support strip.

End portions of the support strip may be at least partially folded over themselves in some examples, creating two layers of material in a region of the commissure toward the leaflets and away from the support structure (e.g., support post 210). In such examples, the secondary sutures 260a and 260b (and secondary sutures 460a and 460b of FIG. 4) may be positioned as shown and extend through both of the above-described layers. Secondary sutures 260a' and 260b' in FIGS. 3A and 3B show example alternative placements for the secondary sutures 260a and 260b, respectively. The alternative secondary sutures 260a' and 260b' pass through only one of the above-described layers of the support strip (e.g., a layer closest to the support post 210). In still other examples, secondary sutures may be positioned in both locations (e.g., a combination of four or three of the sutures 260a, 260b, 260a', and 260b' may be used) in order to provide additional reinforcement of the coupling of the support strip to itself and the commissure tabs 220a and 220b.

The pre-assembled commissure tab assembly of the presently claimed invention, assembled as described in any of the examples above, is attached to a corresponding commissure post 210 (or other commissure support structure) of the frame. The commissure post 210 can be a component of an actuator 80 of the prosthetic valve 10. For example, the upper portion of outer member 92 (FIG. 1) can serve as the commissure post 210. In alternative embodiments, the prosthetic valve 10 can include commissure posts separate from the actuators. The separate commissure posts can be mounted to the inner surface of the frame 12, or can be integral portions of the frame, at locations circumferentially spaced from the actuators.

The commissure tab assembly of the presently claimed invention is attached to the commissure post 210 by extending a tertiary suture 270 through tertiary suture lines 272 formed along the support strip 230 (e.g., each of two opposing sides of the support strip) adjacent two circumferentially opposite sides of the commissure post 210, for example by forming shoelace stitches around the post 210 and knotting both ends of the suture 270 (e.g., forming a knot 278 at the top and/or bottom end). The stitches formed by tertiary suture 270 extend between opposing sides of the support strip across a first side of the post 210, and may optionally extend at least 360 degrees around the post 210.

In some examples, the tertiary suture lines may be spaced from (e.g., and provided in addition to) other suture lines (e.g., primary suture lines, such as lines 452a/452b of FIG. 4, and/or secondary suture lines, such as lines 262a of FIG. 2B and/or lines 462a/462b of FIG. 4). In alternative examples, the tertiary suture lines may at least partially (or, in some examples, fully) overlap or include other suture lines of the support strip (e.g., by utilizing the same apertures and/or by extending in alignment with the other suture line(s), such as having apertures used for tertiary sutures being interspersed with apertures used for primary and/or secondary sutures).

In some examples, as shown in FIG. 3B, the tertiary sutures 270 may only extend through support strip material, such as portions of the support strip that extend beyond an end portion of the commissure tabs 220a and 220b. In other examples, the tertiary sutures 270 may further extend through the commissure tabs (e.g., in addition to extending through the support strip as shown in FIG. 3B). In such examples, the tertiary sutures may pass through one or more layers of the support strip that are adjacent to a first commissure tab (which may be disposed on opposing sides of the first commissure tab), through the first commissure tab, across a surface of the support post 210, through one or more layers of the support strip that are adjacent to a second commissure tab (which may be disposed on opposing sides of the second commissure tab), and through the second commissure tab.

As described above, FIG. 4 shows a primary suture 450 and two secondary sutures 460a and 460b extending between inner secondary suture lines 462a and 462b (respectively) and outer secondary suture lines 464a and 464b (respectively). As shown in FIG. 4, the outer secondary suture lines 464a and 464b are relatively close to each other. This proximity prevents marking holes formation along the positions of suture lines 464a and 464b - as marking holes in such proximity may compromise cloth integrity.

As briefly described above, according to an aspect of the disclosure, there is provided a flexible cloth (e.g., a support strip) comprising a main body portion and a foldable tab extending from one edge of the main body portion, centered therewith, wherein the tab comprises marking holes for the outer secondary suture line.

FIG. 5 shows an example of a support strip 530 that may be used in a commissure of a prosthetic valve of the present disclosure. The support strip 530 may be one example of the support strip 230/430 of FIGS. 2A-4. The support strip 530 comprises a main body portion 532 and a tab 534. The main body portion 532 may be similar in its dimensions to the support strip 230/430 used in FIGS. 2A-4. The tab 534 extends from the lower edge of the main body portion 532 (e.g., at a horizontal fold line of the support strip). The tab can have a length L similar to a width W1 of the main body portion 532. The support strip 530 has a centerline C that includes a centerline of the tab 534 that is continuous with the widthwise centerline of the main body portion 532. The width W2 of the tab 534 may be equal to or slightly smaller than the width of the commissure post or other support structure to which the commissure is to be secured. The support strip 530 can have a T-shape as shown in FIG. 5 prior to folding the tab 534.

The tab 534 further comprises two lines or columns of indicia or markings for the placement of sutures, such as in the form of marking holes 564a and 564b, configured to mark the desired positions of the outer secondary suture lines when the tab 534 is folded over the main body portion 532, as shown in FIG. 6.

According to some embodiments, the main body portion 532 comprises additional indicia or markings for the placement of sutures, such as marking holes 552a and 552b, configured to mark the desired positions of the first suture line and the second suture line formed by the respective marking holes. The columns of marking holes 552a and 552b may be positioned on opposite sides of the tab 534 from one another. In some examples, the marking holes 552a and 552b may be symmetrical with respect to the centerline C of the main body portion 532. The indicia or markings for suture placement described herein may comprise a plurality of pre-formed apertures or openings for receiving sutures (e.g., apertures or openings that extend at least partially through the support strip). In other examples, in lieu of or in addition to apertures, the indicia or markings for suture placement described herein can comprise a plurality of ink markings or other markings that are visible or otherwise provide a visual indication of locations for stitching (e.g., where the markings themselves do not form openings passing through the support strip, but rather indicate where sutures/stitching is to pass through the support strip to assemble the commissure). It is to be understood that the number of indicia for suture placement illustrated herein on the example support strips is exemplary, and any suitable number of stitching lines or other markings may be included on the support strip or other elements of the prosthetic valve (e.g., the commissure tabs, optional reinforcing elements, etc.) without departing from the scope of the disclosure.

According to some embodiments, the main body portion 532 and the tab 534 comprise alignment notches 536 and 538, respectively, along opposing edges thereof. The alignment notches 536 and 538 are similarly shaped and dimensioned and are configured to align with each other when the tab 534 is folded over the main body portion 532 (see FIG. 6), thereby facilitating alignment between the tab 534 and the main body portion 532.

Advantageously, since the tab constitutes an additional cloth portion, not meant to carry load of the commissure assembly, it is possible to add marking holes thereto without compromising cloth integrity once the commissure is assembled and mounted on a post. For example, the marking holes may be provided on the tab instead of the main body such that a region of the main body that is covered by the tab after folding (as shown in FIG. 6) does not include any marking holes. Moreover, the proposed configuration doubles the layer of the cloth having the outer secondary suture lines there along (e.g., where a first layer comprises the main body and a second layer comprises the tab with the marking holes), thereby actually strengthening this cloth layer.

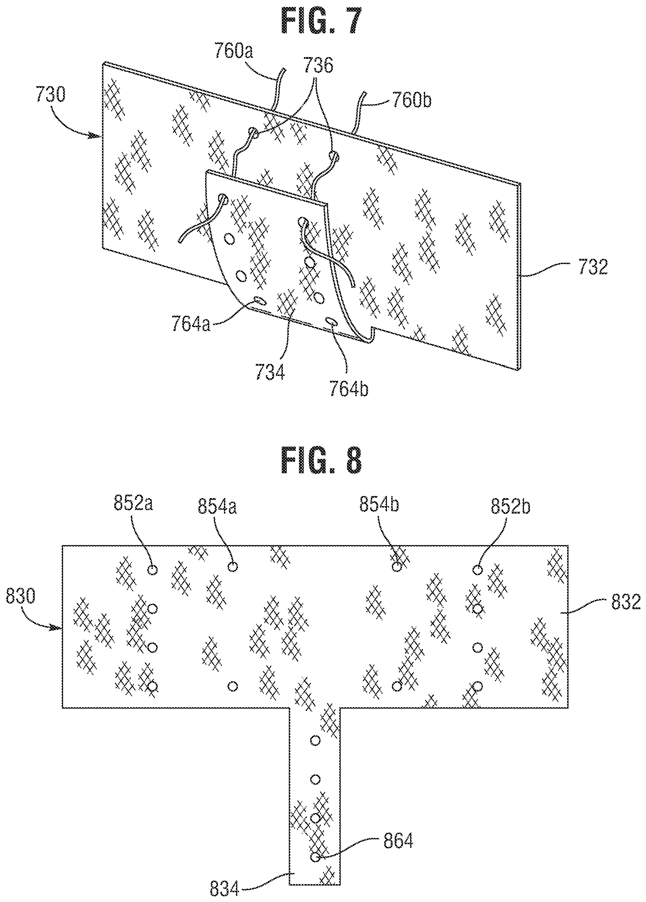

According to some embodiments, the tab comprises a single line or column of markings, such as a single column of marking holes instead of two separate parallel lines of marking holes, configured to accept both outer secondary suture lines formed by marking holes 564a, 564b there along. An example of such a configuration is described below with respect to FIG. 8.

FIG. 7 shows another embodiment of a flexible cloth 730 comprising a main body portion 732 and a tab 734 provided with two lines or columns of indicia or markings in the form of marking holes 764a and 764b. Unlike flexible cloth 530 of FIGS. 5 and 6, the cloth 730 is devoid of alignment notches. Alignment between the tab 734 and the main body portion 732 is alternatively achieved by suturing (e.g., passing sutures 760a and 760b through) alignment apertures 736 with matching apertures along the lines of marking holes 764a and 764b (see FIG. 7), when the tab 734 is folded over the main body portion 732.

In the illustrated example, the main body portion 732 includes only two alignment apertures 736 (e.g., for alignment with corresponding marking holes from each line formed by marking holes 764a and 764b). In other examples, the main body portion 732 may include more or fewer alignment apertures.

FIG. 8 shows another embodiment of a flexible cloth 830 comprising a main body 832 and a tab 834. The main body 832 includes marking hole-lines 852a and 852b and, optionally, supplemental marking hole-lines 854a and 854b. Unlike flexible cloths 530 or 730 of FIGS. 5-6 and 7, respectively, the tab 834 is provided with a single suture line 864. This embodiment allows a suture to extend, when stitched back and forth, through the same holes along the suture line 864.

It is to be understood that any of the above-described example support strips of FIGS. 5-8 may be used in a commissure assembly as described above with respect to FIGS. 2A-4.

Advantageously, since the tab of the present disclosure constitutes an additional cloth portion, which may not be relied upon to carry the load of the commissure assembly, it is possible to add marking holes thereto without compromising cloth integrity once the commissure is assembled and mounted on a post. Moreover, the proposed configuration doubles the layer of the cloth having the outer secondary suture lines there along, thereby actually strengthening this cloth layer.

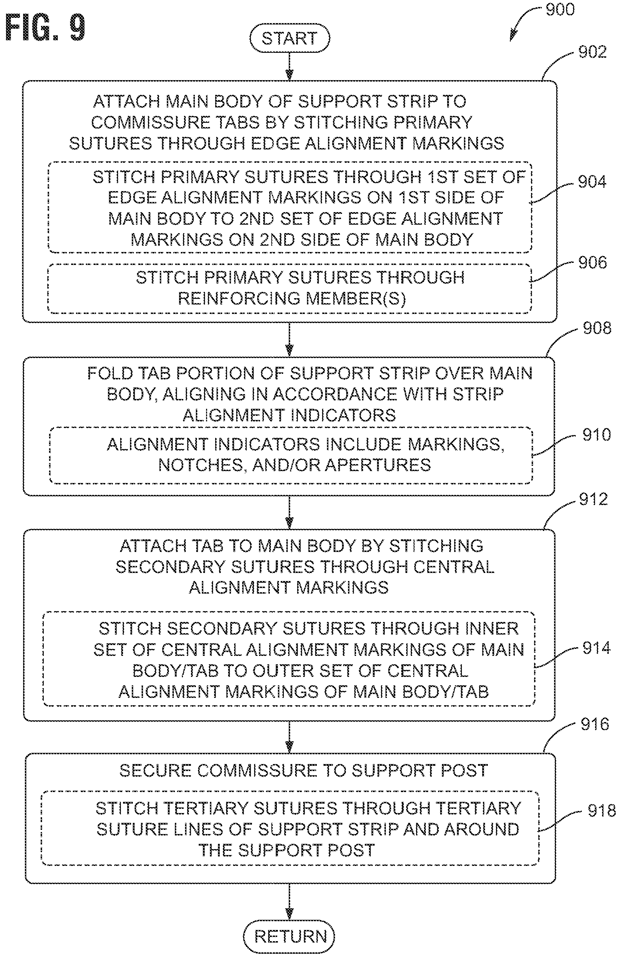

FIG. 9 is a flow chart of an example method 900 of assembling a commissure. For example, method 900 may be performed to assembly any of the example commissures described herein. At 902, the method includes coupling (e.g., securing and/or attaching) a support strip to a pair of commissure tabs (corresponding to a pair of leaflets of a prosthetic valve) by stitching primary sutures through edge alignment markings (e.g., markings 262a/262b and 462a/462b of FIGS. 2A-4) of the support strip.

The support strip may be configured to include a main portion and a tab extending from the main portion (e.g., in a central region of the main portion). For example, the support strip may be configured as described above with respect to support strip 230 of FIGS. 2A-3B, support strip 430 of FIG. 4, support strip 530 of FIGS. 5 and 6, support strip 730 of FIG. 7, and/or support strip 830 of FIG. 8. Accordingly, the main portion of the support strip may include a central region (from which the tab extends) and two opposing side regions disposed on opposite sides of the central region from one another. Securing the support strip to the commissure tabs may include attaching each of the opposing sides of the main portion of the support strip to a respective one of the commissure tabs.

As indicated at 904, the primary sutures may be stitched through a first set of edge alignment markings on a first side of the main body to (and through) a second set of edge alignment markings on a second side of the main body (e.g., on an opposite side of the main body from the first side). Example edge alignment markings are shown at 552a/552b of FIGS. 5 and 6 and 852a/852b of FIG. 8. Suture lines 452a/452b of FIG. 4 may also be formed of markings that correspond to example edge alignment markings.

In some examples, where reinforcing members are used, such as reinforcing elements 240a and 240b illustrated in FIGS. 2A and 2B and/or reinforcing elements 440a and 440b of FIG. 4, the primary sutures may also extend through the reinforcing members, as indicated at 906.

At 908, the method includes folding a tab portion of the support strip over the main body of the support strip, aligning in accordance with strip alignment indicators. The tab portion may be folded at a horizontal fold line such that the tab portion overlaps a center portion of the main body of the support strip. For example, the support strip may be T-shaped before folding. In some examples, the height of the main body of the support strip (e.g., the top, horizontal portion of the T-shape) may be smaller than or the same as the height of the tab portion (e.g., the bottom, vertical portion of the T-shape). In other examples, the height of the main body of the support strip may be larger than the height of the tab portion. In the any of the above described examples, the width of the tab portion of the support strip may be smaller than or the same as the width of the main body. After folding the tab portion over the central portion of the main body, the support strip may be substantially rectangular. As indicated at 910, the strip alignment indicators may include markings, notches, and/or apertures. Examples of markings and/or apertures are shown at 564a/564b of FIGS. 5 and 6, 764a/764b of FIG. 7, and 864 of FIG. 8. Examples of notches are shown at 536/538 of FIGS. 5 and 6.

At 912, the method includes attaching the tab portion of the support strip to the main body by stitching secondary sutures through central alignment markings. In some examples, the central alignment markings may include one or more of the strip alignment indicators (e.g., in examples where the strip alignment indicators include markings and/or apertures, the strip alignment markings and/or apertures may also be used as at least some of the central alignment markings. Examples of central alignment markings may include one or more of markings 564a/564b of FIGS. 5 and 6, markings 736 and 764a/764b of FIG. 7, and markings 854a/854b and/or 864 of FIG. 8. Suture lines through which sutures 260a/260b of FIGS. 2A-3B pass and suture lines 462a/462b and 464a/464b of FIG. 4 may also be formed of markings that correspond to example edge alignment markings.

As indicated at 914, the attachment may include stitching the secondary sutures through an inner set of central alignment markings of the main body and/or tab portion of the support structure to (and through) an outer set of central alignment markings of the main body and/or tab portion of the support strip. For example, suture lines 262a/262b of FIGS. 2A-3B (or correspondingly suture lines 462a/462b of FIG. 4) may be formed of markings that are examples of an inner set of central alignment markings, while suture lines 264a/264b of FIGS. 2A-3B (or correspondingly suture lines 464a/464b of FIG. 4) may be formed of markings that are examples of an outer set of central alignment markings (or vice versa - 262a/262b/462a/462b may correspond to the outer set while 264a/264b/464a/464b may correspond to the inner set).

At 916, the method includes securing the commissure to a support post and/or another element of a frame of the prosthetic valve in which the commissure is disposed. As indicated at 918, securing the commissure to the support post may include stitching tertiary sutures through tertiary suture lines of the support strip and around the support post. Example tertiary sutures are shown at 270 and example tertiary suture lines are shown at 272 in FIGS. 2A-3B. As further shown in FIG. 2A, the securing of the commissure to the support post may further include coupling end portions of the tertiary suture to one another (e.g., via a knot, such as knot 278). In some examples, one or more of the markings of the support strip examples of FIGS. 5-8 and/or additional markings of the support strip may be used to mark tertiary suture lines.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims. We therefore claim as our invention all that comes within the scope of these claims.

## Claims

1. A method of assembling a commissure of a prosthetic heart valve, the method comprising:
pre-assembling a commissure tab assembly, wherein the pre-assembling comprises:
extending a primary suture (250) along a first primary suture line to form a plurality of in-and-out stitches extending through, in order or in reverse order, a first side of a support strip (230), a first commissure tab (220a) of a first leaflet (221a), a second commissure tab (220b) of a second leaflet (221b), and along a second primary suture line on a second side of the support strip;
extending a first secondary suture (260a), in order or reverse order, between a first inner secondary suture line (262a), through the support strip, through the first commissure tab and along a first outer secondary suture line (264a) of a mid-portion of the support strip; and
extending a second secondary suture (260b), in order or reverse order, between a second inner secondary suture line (262b), through the support strip, through the second commissure tab and along a second outer secondary suture line (264b) of the mid-portion of the support strip; and
attaching the pre-assembled commissure tab assembly to a commissure post (210) of a frame of a prosthetic heart valve by extending a tertiary suture (270) through tertiary suture lines (272) formed along the support strip adjacent two circumferentially opposite sides of the commissure post, such that the stitches formed by the tertiary suture extend between opposing sides of the support strip across a first side of the commissure post;
wherein the first commissure tab and the second commissure tab are two commissure tabs of adjacent leaflets (221a and 221b) of a prosthetic heart valve; and
wherein the second side of the support strip is opposite the first side of the support strip relative to a centre of the support strip.

2. A prosthetic heart valve comprising:
a frame comprising commissure posts (210);
a plurality of leaflets (221a, 221b);
and
commissure tab assemblies, each commissure tab assembly comprising;
a primary suture (250) extending along a first primary suture line to form a plurality of in-and-out stitches extending through, in order or in reverse order, a first side of a support strip (230), a first commissure tab (220a) of a first leaflet (221a), a second commissure tab (220b) of a second leaflet (221b), and along a second primary suture line on a second side of the support strip;
a first secondary suture (260a) extending, in order or reverse order, between a first inner secondary suture line (262a), through the support strip, through the first commissure tab and along a first outer secondary suture line (264a) of a mid-portion of the support strip;
a second secondary suture (260b) extending, in order or reverse order, between a second inner secondary suture line (262b), through the support strip, through the second commissure tab and along a second outer secondary suture line (264b) of the mid-portion of the support strip;
wherein each commissure tab assembly is attached to a commissure post (210) of the frame by a tertiary suture (270) extending through tertiary suture lines (272) formed along the support strip adjacent two circumferentially opposite sides of the commissure post, such that the stitches formed by the tertiary suture extend between opposing sides of the support strip across a first side of the commissure post;
wherein the first commissure tab and the second commissure tab are two commissure tabs of adjacent leaflets (221a and 221b) of a prosthetic heart valve; and
wherein the second side of the support strip is opposite the first side of the support strip relative to a centre of the support strip.

3. The method of claim 1 or the prosthetic heart valve of claim 2, wherein the tertiary suture lines are spaced from the first primary suture line, second primary suture line, first inner secondary suture line, first outer secondary suture line, second inner secondary suture line and the second outer secondary suture line.

4. The method of claim 1 or claim 3, or the prosthetic heart valve of claim 2 or claim 3, wherein the tertiary sutures only extend through the support strip,
wherein the tertiary sutures only extend through portions of the support strip that extend beyond an end portion of the first and second commissure tabs.

5. The method of claim 1 or claim 3, or the prosthetic heart valve of claim 2 or claim 3, wherein the tertiary sutures extend through the support strip and the commissure tabs,
wherein the tertiary sutures pass through one or more layers of the support strip that are adjacent to the first commissure tab, through the first commissure tab, across a surface of the commissure post, through one or more layers of the support strip that are adjacent to the second commissure tab and through the second commissure tab.

6. The method of any of claims 1 and 3 to 5, or the prosthetic heart valve of any of claims 2 to 5, wherein the tertiary suture (270) comprises
shoelace stiches formed around the commissure post and knotted at both ends of the tertiary suture,
optionally wherein the stitches extend at least 360 degrees around the commissure post.

7. The method of any of claims 1 and 3 to 6, or the prosthetic heart valve of any of claims 2 to 6, wherein the prosthetic heart valve comprises a plurality of actuators (80) mounted to and equally spaced around an inner surface of the frame, wherein the plurality of actuators are configured to apply expansion and compression to the frame for radially expanding and compressing the prosthetic valve and wherein each commissure post comprises a component of one of the actuators.

8. The method of any of claims 1 and 3 to 6, or the prosthetic heart valve of any of claims 2 to 6, wherein the prosthetic heart valve comprises a plurality of actuators (80) mounted to and equally spaced around an inner surface of the frame, wherein the plurality of actuators are configured to apply expansion and compression to the frame for radially expanding and compressing the prosthetic valve and wherein the commissure post is separate from the actuators,
optionally wherein the commissure post:
a) is mounted to the inner surface of the frame at a location circumferentially spaced from the actuators; or
b) comprises an integral portion of the frame, at a location circumferentially spaced from the actuators.

9. The method of any of claims 1 and 3 to 8, or the prosthetic heart valve of any of claims 2 to 8, wherein the commissure tab assembly additionally comprises a first reinforcing element (240a) and a second reinforcing element (240b) and wherein the primary suture extends through, in order or in reverse order, the first side of the support strip, the first reinforcing element, the first commissure tab of the first leaflet, the second commissure tab of the second leaflet, the second reinforcing element, and along the second suture line on the second side of the support strip.

10. The method or prosthetic heart valve of claim 9, wherein the first reinforcing element and second reinforcing element comprise:
a) two individual reinforcing elements; or
b) two portions of a single reinforcing member that extends along outer surfaces of each of the first and second commissure tabs, optionally wherein the reinforcing member is folded into a U-shape configuration to form the reinforcing elements.

11. The method or prosthetic heart valve of claim 9 or 10, wherein the first reinforcing element is sandwiched between an outer surface of the first commissure tab and a first end or side portion of the support strip, and the second reinforcing element is sandwiched between an outer surface of the second commissure tab and a second end or side portion of the support strip.

12. The method or prosthetic heart valve of any of claims 9 to 11, where the first secondary suture extends, in order or reverse order, between the first inner secondary suture line, through the support strip, through the first reinforcing element, through the first commissure tab and along the first outer secondary suture line of the mid-portion of the support strip; and wherein the second secondary suture extends, in order or reverse order, between the second inner secondary suture line, through the support strip, through the second reinforcing element, through the second commissure tab and along the second outer secondary suture line of the mid-portion of the support strip.

13. The method of any of claims 1 and 3 to 12, and the prosthetic heart valve of any of claims 2 to 12, wherein a first end portion of the support strip and a second end portion of the support strip are each at least partially folded over themselves to create two layers of material in a region of the commissure towards the leaflets and away from the commissure post,
optionally wherein:
a) the first secondary suture extends through both of the two layers of material at the first end portion of the support strip, and the second secondary suture extends through both of the two layers of material at the second end portion of the support strip; or
b) the first secondary suture extends through only one layer of material at the first end portion of the support strip, and the second secondary suture extends through only one layer of material at the second end portion of the support strip, wherein the one layer comprises the layer closest to the commissure post.

## Patentansprüche

1. Verfahren zum Herstellen einer Kommissur einer Herzklappenprothese, wobei das Verfahren Folgendes umfasst:
vorheriges Herstellen einer Kommissurlascheneinheit, wobei das vorherige Herstellen Folgendes umfasst:
Führen einer Primärnaht (250) entlang einer ersten Primärnahtlinie, um eine Vielzahl von Ein- und Ausstichen zu bilden, die sich der Reihenfolge entsprechend oder in umgekehrter Reihenfolge durch eine erste Seite eines Stützstreifens (230), eine erste Kommissurlasche (220a) eines ersten Segels (221a), eine zweite Kommissurlasche (220b) eines zweiten Segels (221b) und entlang einer zweiten Primärnahtlinie auf einer zweiten Seite des Stützstreifens erstrecken;
Führen einer ersten Sekundärnaht (260a) der Reihenfolge entsprechend oder in umgekehrter Reihenfolge zwischen einer ersten inneren Sekundärnahtlinie (262a), durch den Stützstreifen, durch die erste Kommissurlasche und entlang einer ersten äußeren Sekundärnahtlinie (264a) eines Mittelabschnitts des Stützstreifens; und
Führen einer zweiten Sekundärnaht (260b) der Reihenfolge entsprechend oder in umgekehrter Reihenfolge zwischen einer zweiten inneren Sekundärnahtlinie (262b), durch den Stützstreifen, durch die zweite Kommissurlasche und entlang einer zweiten äußeren Sekundärnahtlinie (264b) des Mittelabschnitts des Stützstreifens; und
Anbringen der vorher hergestellten Kommissurlascheneinheit an einem Kommissurpfosten (210) eines Rahmens einer Herzklappenprothese durch Führen einer Tertiärnaht (270) durch Tertiärnahtlinien (272), die entlang des Stützstreifens neben zwei in Umfangsrichtung gegenüberliegenden Seiten des Kommissurpfostens ausgebildet sind, so dass sich die durch die Tertiärnaht gebildeten Stiche zwischen gegenüberliegenden Seiten des Stützstreifens über eine erste Seite des Kommissurpfostens erstrecken;
wobei die erste Kommissurlasche und die zweite Kommissurlasche zwei Kommissurlaschen benachbarter Segel (221a und 221b) einer Herzklappenprothese sind; und
wobei die zweite Seite des Stützstreifens der ersten Seite des Stützstreifens relativ zu einer Mitte des Stützstreifens gegenüberliegt.

2. Herzklappenprothese, die Folgendes umfasst:
einen Rahmen, der Kommissurpfosten (210) umfasst;
eine Vielzahl von Segeln (221a, 221b);
und
Kommissurlascheneinheiten, wobei jede Kommissurlascheneinheit Folgendes umfasst:
eine Primärnaht (250), die sich entlang einer ersten Primärnahtlinie erstreckt, um eine Vielzahl von Ein- und Ausstichen zu bilden, die sich der Reihenfolge entsprechend oder in umgekehrter Reihenfolge durch eine erste Seite eines Stützstreifens (230), eine erste Kommissurlasche (220a) eines ersten Segels (221a), eine zweite Kommissurlasche (220b) eines zweiten Segels (221b) und entlang einer zweiten Primärnahtlinie auf einer zweiten Seite des Stützstreifens erstrecken;
eine erste Sekundärnaht (260a), die sich der Reihenfolge entsprechend oder in umgekehrter Reihenfolge zwischen einer ersten inneren Sekundärnahtlinie (262a), durch den Stützstreifen, durch die erste Kommissurlasche und entlang einer ersten äußeren Sekundärnahtlinie (264a) eines Mittelabschnitts des Stützstreifens erstreckt;
eine zweite Sekundärnaht (260b), die sich der Reihenfolge entsprechend oder in umgekehrter Reihenfolge zwischen einer zweiten inneren Sekundärnahtlinie (262b), durch den Stützstreifen, durch die zweite Kommissurlasche und entlang einer zweiten äußeren Sekundärnahtlinie (264b) des Mittelabschnitts des Stützstreifens erstreckt;
wobei jede Kommissurlascheneinheit durch eine Tertiärnaht (270), die sich durch Tertiärnahtlinien (272), die entlang des Stützstreifens neben zwei in Umfangsrichtung gegenüberliegenden Seiten des Kommissurpfostens ausgebildet sind, an einem Kommissurpfosten (210) des Rahmens angebracht ist, so dass sich die durch die Tertiärnaht gebildeten Stiche zwischen gegenüberliegenden Seiten des Stützstreifens über eine erste Seite des Kommissurpfostens erstrecken;
wobei die erste Kommissurlasche und die zweite Kommissurlasche zwei Kommissurlaschen benachbarter Segel (221a und 221b) einer Herzklappenprothese sind; und
wobei die zweite Seite des Stützstreifens der ersten Seite des Stützstreifens relativ zu einer Mitte des Stützstreifens gegenüberliegt.

3. Verfahren nach Anspruch 1 oder Herzklappenprothese nach Anspruch 2, wobei die Tertiärnahtlinien von der ersten Primärnahtlinie, der zweiten Primärnahtlinie, der ersten inneren Sekundärnahtlinie, der ersten äußeren Sekundärnahtlinie, der zweiten inneren Sekundärnahtlinie und der zweiten äußeren Sekundärnahtlinie beabstandet sind.

4. Verfahren nach Anspruch 1 oder Anspruch 3 oder Herzklappenprothese nach Anspruch 2 oder Anspruch 3, wobei sich die Tertiärnähte nur durch den Stützstreifen erstrecken,
wobei sich die Tertiärnähte nur durch Abschnitte des Stützstreifens erstrecken, die sich über einen Endabschnitt der ersten und der zweiten Kommissurlasche hinaus erstrecken.

5. Verfahren nach Anspruch 1 oder Anspruch 3 oder Herzklappenprothese nach Anspruch 2 oder Anspruch 3, wobei sich die Tertiärnähte durch den Stützstreifen und die Kommissurlaschen erstrecken,
wobei die Tertiärnähte durch eine oder mehrere Schichten des Stützstreifens, die an die erste Kommissurlasche angrenzen, durch die erste Kommissurlasche, über eine Oberfläche des Kommissurpfostens, durch eine oder mehrere Schichten des Stützstreifens, die an die zweite Kommissurlasche angrenzen, und durch die zweite Kommissurlasche verlaufen.

6. Verfahren nach einem der Ansprüche 1 und 3 bis 5 oder Herzklappenprothese nach einem der Ansprüche 2 bis 5, wobei die Tertiärnaht (270) Folgendes umfasst:
Schnürstiche, die um den Kommissurpfosten herum gebildet und an beiden Enden der Tertiärnaht geknotet sind,
wobei sich die Stiche optional um mindestens 360 Grad um den Kommissurpfosten herum erstrecken.

7. Verfahren nach einem der Ansprüche 1 und 3 bis 6 oder Herzklappenprothese nach einem der Ansprüche 2 bis 6, wobei die Herzklappenprothese eine Vielzahl von Aktuatoren (80) umfasst, die an einer Innenfläche des Rahmens montiert und gleichmäßig um diese herum beabstandet sind, wobei die Vielzahl von Aktuatoren dazu ausgelegt ist, Expansion und Kompression auf den Rahmen zum radialen Expandieren und Komprimieren der Klappenprothese anzuwenden, und wobei jeder Kommissurpfosten eine Komponente eines der Aktuatoren umfasst.

8. Verfahren nach einem der Ansprüche 1 und 3 bis 6 oder Herzklappenprothese nach einem der Ansprüche 2 bis 6, wobei die Herzklappenprothese eine Vielzahl von Aktuatoren (80) umfasst, die an einer Innenfläche des Rahmens montiert und gleichmäßig um diese herum beabstandet sind, wobei die Vielzahl von Aktuatoren dazu ausgelegt ist, Expansion und Kompression auf den Rahmen zum radialen Expandieren und Komprimieren der Klappenprothese anzuwenden, und wobei der Kommissurpfosten von den Aktuatoren getrennt ist,
wobei der Kommissurpfosten optional:
a) an der Innenfläche des Rahmens an einer in Umfangsrichtung von den Aktuatoren beabstandeten Stelle montiert ist; oder
b) einen integralen Abschnitt des Rahmens an einer in Umfangsrichtung von den Aktuatoren beabstandeten Stelle umfasst.

9. Verfahren nach einem der Ansprüche 1 und 3 bis 8 oder Herzklappenprothese nach einem der Ansprüche 2 bis 8, wobei die Kommissurlascheneinheit zusätzlich ein erstes Verstärkungselement (240a) und ein zweites Verstärkungselement (240b) umfasst und wobei sich die Primärnaht der Reihenfolge entsprechend oder in umgekehrter Reihenfolge durch die erste Seite des Stützstreifens, das erste Verstärkungselement, die erste Kommissurlasche des ersten Segels, die zweite Kommissurlasche des zweiten Segels, das zweite Verstärkungselement, und entlang der zweiten Nahtlinie auf der zweiten Seite des Stützstreifens erstreckt.

10. Verfahren oder Herzklappenprothese nach Anspruch 9, wobei das erste Verstärkungselement und das zweite Verstärkungselement Folgendes umfassen:
a) zwei individuelle Verstärkungselemente; oder
b) zwei Abschnitte eines einzelnen Verstärkungselements, die sich entlang Außenflächen jeder der ersten und der zweiten Kommissurlasche erstrecken, wobei das Verstärkungselement optional in eine U-förmige Konfiguration geklappt ist, um die Verstärkungselemente zu bilden.

11. Verfahren oder Herzklappenprothese nach Anspruch 9 oder 10, wobei das erste Verstärkungselement zwischen einer Außenfläche der ersten Kommissurlasche und einem ersten Ende oder Seitenabschnitt des Stützstreifens eingeschoben ist und das zweite Verstärkungselement zwischen einer Außenfläche der zweiten Kommissurlasche und einem zweiten Ende oder Seitenabschnitt des Stützstreifens eingeschoben ist.

12. Verfahren oder Herzklappenprothese nach einem der Ansprüche 9 bis 11, wobei sich die erste Sekundärnaht der Reihenfolge entsprechend oder in umgekehrter Reihenfolge zwischen der ersten inneren Sekundärnahtlinie, durch den Stützstreifen, durch das erste Verstärkungselement, durch die erste Kommissurlasche und entlang der ersten äußeren Sekundärnahtlinie des Mittelabschnitts des Stützstreifens erstreckt und wobei sich die zweite Sekundärnaht der Reihenfolge entsprechend oder in umgekehrter Reihenfolge zwischen der zweiten inneren Sekundärnahtlinie, durch den Stützstreifen, durch das zweite Verstärkungselement, durch die zweite Kommissurlasche und entlang der zweiten äußeren Sekundärnahtlinie des Mittelabschnitts des Stützstreifens erstreckt.

13. Verfahren nach einem der Ansprüche 1 und 3 bis 12 und Herzklappenprothese nach einem der Ansprüche 2 bis 12, wobei ein erster Endabschnitt des Stützstreifens und ein zweiter Endabschnitt des Stützstreifens jeweils zumindest teilweise über sich selbst geklappt sind, um zwei Materialschichten in einem Bereich der Kommissur zu den Segeln hin und von dem Kommissurpfosten weg zu erzeugen,
wobei optional:
a) die erste Sekundärnaht sich durch beide der zwei Materialschichten an dem ersten Endabschnitt des Stützstreifens erstreckt und die zweite Sekundärnaht sich durch beide der zwei Materialschichten an dem zweiten Endabschnitt des Stützstreifens erstreckt; oder
b) die erste Sekundärnaht sich durch nur eine Materialschicht an dem ersten Endabschnitt des Stützstreifens erstreckt und die zweite Sekundärnaht sich durch nur eine Materialschicht an dem zweiten Endabschnitt des Stützstreifens erstreckt, wobei die eine Schicht die am nächsten bei dem Kommissurpfosten liegende Schicht umfasst.

## Revendications

1. Procédé d'assemblage d'une commissure d'une valvule cardiaque prothétique, le procédé comprenant :
le pré-assemblage d'un ensemble de languette de commissure, le pré-assemblage comprenant :
l'extension d'une suture primaire (250) le long d'une première ligne de suture primaire pour former une pluralité de points d'entrée et de sortie s'étendant à travers, dans l'ordre ou dans l'ordre inverse, un premier côté d'une bande de support (230), une première languette de commissure (220a) d'un premier feuillet (221a), une seconde languette de commissure (220b) d'un second feuillet (221b), et le long d'une seconde ligne de suture primaire sur un second côté de la bande de support ;
l'extension d'une première suture secondaire (260a), dans l'ordre ou dans l'ordre inverse, entre une première ligne de suture secondaire interne (262a), à travers la bande de support, à travers la première languette de commissure et le long d'une première ligne de suture secondaire externe (264a) d'une partie médiane de la bande de support ; et
l'extension d'une seconde suture secondaire (260b), dans l'ordre ou dans l'ordre inverse, entre une seconde ligne de suture secondaire interne (262b), à travers la bande de support, à travers la seconde languette de commissure et le long d'une seconde ligne de suture secondaire externe (264b) de la partie médiane de la bande de support ; et
la fixation de l'ensemble de languette de commissure pré-assemblé à un montant de commissure (210) d'un cadre d'une valvule cardiaque prothétique en étendant une suture tertiaire (270) à travers des lignes de suture tertiaires (272) formées le long de la bande de support adjacentes à deux côtés circonférentiellement opposés du montant de commissure, de telle sorte que les points formés par la suture tertiaire s'étendent entre des côtés opposés de la bande de support sur un premier côté du montant de commissure ;
la première languette de commissure et la seconde languette de commissure étant deux languettes de commissure de feuillets adjacents (221a et 221b) d'une valvule cardiaque prothétique ; et
le second côté de la bande de support étant opposé au premier côté de la bande de support par rapport à un centre de la bande de support.

2. Valvule cardiaque prothétique comprenant :
un cadre comprenant des montants de commissure (210) ;
une pluralité de feuillets (221a, 221b) ;
et
des ensembles de languette de commissure, chaque ensemble de languette de commissure comprenant :
une suture primaire (250) s'étendant le long d'une première ligne de suture primaire pour former une pluralité de points d'entrée et de sortie s'étendant à travers, dans l'ordre ou dans l'ordre inverse, un premier côté d'une bande de support (230), une première languette de commissure (220a) d'un premier feuillet (221a), une seconde languette de commissure (220b) d'un second feuillet (221b), et le long d'une seconde ligne de suture primaire sur un second côté de la bande de support ;
une première suture secondaire (260a) s'étendant, dans l'ordre ou dans l'ordre inverse, entre une première ligne de suture secondaire interne (262a), à travers la bande de support, à travers la première languette de commissure et le long d'une première ligne de suture secondaire externe (264a) d'une partie médiane de la bande de support ;
une seconde suture secondaire (260b) s'étendant, dans l'ordre ou dans l'ordre inverse, entre une seconde ligne de suture secondaire interne (262b), à travers la bande de support, à travers la seconde languette de commissure et le long d'une seconde ligne de suture secondaire externe (264b) de la partie médiane de la bande de support ;
chaque ensemble de languette de commissure étant fixé à un montant de commissure (210) du cadre par une suture tertiaire (270) s'étendant à travers des lignes de suture tertiaires (272) formées le long de la bande de support adjacentes à deux côtés circonférentiellement opposés du montant de commissure, de telle sorte que les points formés par la suture tertiaire s'étendent entre des côtés opposés de la bande de support sur un premier côté du montant de commissure ;
la première languette de commissure et la seconde languette de commissure étant deux languettes de commissure de feuillets adjacents (221a et 221b) d'une valvule cardiaque prothétique ; et
le second côté de la bande de support étant opposé au premier côté de la bande de support par rapport à un centre de la bande de support.

3. Procédé selon la revendication 1 ou valvule cardiaque prothétique selon la revendication 2, les lignes de suture tertiaires étant espacées de la première ligne de suture primaire, de la seconde ligne de suture primaire, de la première ligne de suture secondaire interne, de la première ligne de suture secondaire externe, de la seconde ligne de suture secondaire interne et de la seconde ligne de suture secondaire externe.

4. Procédé selon la revendication 1 ou la revendication 3, ou valvule cardiaque prothétique selon la revendication 2 ou la revendication 3, les sutures tertiaires s'étendant uniquement à travers la bande de support,
les sutures tertiaires s'étendant uniquement à travers des parties de la bande de support qui s'étendent au-delà d'une partie d'extrémité des première et seconde languettes de commissure.

5. Procédé selon la revendication 1 ou la revendication 3, ou valvule cardiaque prothétique selon la revendication 2 ou la revendication 3, les sutures tertiaires s'étendant à travers la bande de support et les languettes de commissure,
les sutures tertiaires passant à travers une ou plusieurs couches de la bande de support qui sont adjacentes à la première languette de commissure, à travers la première languette de commissure, sur une surface du montant de commissure, à travers une ou plusieurs couches de la bande de support qui sont adjacentes à la seconde languette de commissure et à travers la seconde languette de commissure.

6. Procédé selon l'une quelconque des revendications 1 et 3 à 5, ou valvule cardiaque prothétique selon l'une quelconque des revendications 2 à 5, la suture tertiaire (270) comprenant
des points en lacet formés autour du montant de commissure et noués aux deux extrémités de la suture tertiaire,
éventuellement les points s'étendant sur au moins 360 degrés autour du montant de commissure.

7. Procédé selon l'une quelconque des revendications 1 et 3 à 6, ou valvule cardiaque prothétique selon l'une quelconque des revendications 2 à 6, la valvule cardiaque prothétique comprenant une pluralité d'actionneurs (80) montés sur une surface interne du cadre et espacés de manière égale autour de celle-ci, la pluralité d'actionneurs étant configurés pour appliquer une expansion et une compression au cadre pour dilater et comprimer radialement la valvule prothétique, et chaque montant de commissure comprenant un composant de l'un des actionneurs.

8. Procédé selon l'une quelconque des revendications 1 et 3 à 6, ou valvule cardiaque prothétique selon l'une quelconque des revendications 2 à 6, la valvule cardiaque prothétique comprenant une pluralité d'actionneurs (80) montés sur une surface interne du cadre et espacés de manière égale autour de celle-ci, la pluralité d'actionneurs étant configurés pour appliquer une expansion et une compression au cadre pour dilater et comprimer radialement la valvule prothétique, et le montant de commissure étant séparé des actionneurs, éventuellement le montant de commissure :
a) étant monté sur la surface interne du cadre à un emplacement circonférentiellement espacé des actionneurs ; ou
b) comprenant une partie intégrante du cadre, à un emplacement circonférentiellement espacé des actionneurs.

9. Procédé selon l'une quelconque des revendications 1 et 3 à 8, ou valvule cardiaque prothétique selon l'une quelconque des revendications 2 à 8, l'ensemble de languette de commissure comprenant en outre un premier élément de renforcement (240a) et un second élément de renforcement (240b), et la suture primaire s'étendant à travers, dans l'ordre ou dans l'ordre inverse, le premier côté de la bande de support, le premier élément de renforcement, la première languette de commissure du premier feuillet, la seconde languette de commissure du second feuillet, le second élément de renforcement, et le long de la seconde ligne de suture sur le second côté de la bande de support.

10. Procédé ou valvule cardiaque prothétique selon la revendication 9, le premier élément de renforcement et le second élément de renforcement comprenant :
a) deux éléments de renforcement individuels ; ou
b) deux parties d'un seul élément de renforcement qui s'étend le long de surfaces externes de chacune des première et seconde languettes de commissure, éventuellement l'élément de renforcement étant plié selon une configuration en U pour former les éléments de renforcement.

11. Procédé ou valvule cardiaque prothétique selon la revendication 9 ou 10, le premier élément de renforcement étant intercalé entre une surface externe de la première languette de commissure et une première partie d'extrémité ou partie latérale de la bande de support, et le second élément de renforcement étant intercalé entre une surface externe de la seconde languette de commissure et une seconde partie d'extrémité ou partie latérale de la bande de support.

12. Procédé ou valvule cardiaque prothétique selon l'une quelconque des revendications 9 à 11, la première suture secondaire s'étendant, dans l'ordre ou dans l'ordre inverse, entre la première ligne de suture secondaire interne, à travers la bande de support, à travers le premier élément de renforcement, à travers la première languette de commissure et le long de la première ligne de suture secondaire externe de la partie médiane de la bande de support ; et la seconde suture secondaire s'étendant, dans l'ordre ou dans l'ordre inverse, entre la seconde ligne de suture secondaire interne, à travers la bande de support, à travers le second élément de renforcement, à travers la seconde languette de commissure et le long de la seconde ligne de suture secondaire externe de la partie médiane de la bande de support.

13. Procédé selon l'une quelconque des revendications 1 et 3 à 12, et valvule cardiaque prothétique selon l'une quelconque des revendications 2 à 12, une première partie d'extrémité de la bande de support et une seconde partie d'extrémité de la bande de support étant chacune au moins partiellement repliées sur elles-mêmes pour créer deux couches de matériau dans une région de la commissure orientée vers les feuillets et à l'écart du montant de commissure,
éventuellement :
a) la première suture secondaire s'étendant à travers les deux couches de matériau au niveau de la première partie d'extrémité de la bande de support, et la seconde suture secondaire s'étendant à travers les deux couches de matériau au niveau de la seconde partie d'extrémité de la bande de support ; ou
b) la première suture secondaire s'étendant à travers une seule couche de matériau au niveau de la première partie d'extrémité de la bande de support, et la seconde suture secondaire s'étendant à travers une seule couche de matériau au niveau de la seconde partie d'extrémité de la bande de support, ladite couche comprenant la couche la plus proche du montant de commissure.
